Europäisches Patentamt

European Patent Office

Office européen des brevets

(11) Publication number: **0 109 373**
A2

(12) # EUROPEAN PATENT APPLICATION

(21) Application number: 83850304.3

(22) Date of filing: 11.11.83

(51) Int. Cl.³: **A 61 B 5/00**, G 01 F 3/24

(30) Priority: **12.11.82 SE 8206430**

(71) Applicant: **Aktiebolaget Meteve, Humlegardsgatan 3, S-412 74 Göteborg (SE)**

(43) Date of publication of application: **23.05.84**
**Bulletin 84/21**

(72) Inventor: **Ekbladh, Fred Vage Gunnar, Rosengatan 9, S-434 00 Kungsbacka (SE)**
Inventor: **Tillander, Hans, Humlegardsgatan 3, S-412 74 Göteborg (SE)**

(84) Designated Contracting States: **AT BE CH DE FR GB IT LI LU NL SE**

(74) Representative: **Inger, Lars Ulf Bosson, L + U INGER Patentbyra HB Garvaregatan 12, S-262 00 Ängelholm (SE)**

(54) **Device for measuring urine.**

(57) The present invention relates to a device for the measurement of urine excreted from a patient being provided with a catheter, whereby it comprises a vertical tube (2) connected via a connector (9) to said catheter; at least a photocell/light source unit (6, 7) arranged around said tube (2) and arranged to close, and open, respectively, the lower end of the vertical tube, whereby this control can be registered and calculated in any desired manner.

EP 0 109 373 A2

DEVICE FOR MEASURING URINE          0109373

Description

Technical Field

The present invention relates to a device for measuring urine, particularly for measuring the urine excreted by a catherized patient.

The object of the present invention is to obtain a possibility to measure small quantities of urine excreted by a patient, whereby the measurement shall be carried out in a safe way and for the caretaking personal in a simple and rational way.

Background of the invention

At incisions in the urine bladder and the urine ducts and at more severe illnesses there is a need for measuring the amount of urine excreted in order to be able to check if the incision has been sucessful, if the kidney function is normal, etc. Thus amounts of urine excreted decreasing to some millilitres per hour is a clear indication of uremia (urine poisoning) is on its way or can easily be on its way. One has thus previously proposed, and used different measuring beakers connected to a urine catheter, often a Foley-catheter, whereby the measuring beakers/devices have been divided into different chambers to allow measurement of small and larger volumes of urine excreted. The staff has therby to measure the flow per time unit "with the clock in the hand", whereby the time unit can be one or some minutes up to some hours dependent on the condition of the patient. The urine meter shall thereby, at every measurement or in each case from time to time be emptied, which is done by opening some bottom valves or by turning over the urine meter so that the urine flows down into a larger collecting container, most often a so called urine bag. This system has mow turned out to be most unsatisfactoryas it calls for an intense attention by the staff. Thus the staff has to come up to the patient´s bed and note the amount of urine excreted, relatively often, and in situation of crisis, very often. Further valves shall be opened

2                    0109373

at emptying or the measuring beaker be turned over, which makes an extra action, and particularly in the latter case is a risk in that connecting tubes loosen with the accompanying risk for infections to and fro the environment.

It has also turned out that one loses certain measure points depending on other work tasks and that measure values are lost when shifting staff.

It has thus been requested a urine measuring device which gives accurate measure values, and which is simple and rational to handle and requests a minimum of supervision.

Description of the present invention

It has now surprisingly been found possible to solve the above mentioned problems by means of the present invention which is characterized in a vertically arranged tube arranged to be connected with one connector arranged in its side to said catheter; at least one photocell, and one to said photocell related light source, respectively, arranged around the upper part of the tube, and a closing device for closing the lower outlet end of the tube or closing a part connected to the tube.

Further characteristics of the invemtion are evident from the accompanying claims.

The invention will be more closely described in the following with reference to the attached drawing, wherein

Fig 1    shows a schematic view of a preferred embodiment of the invention;

Fig 2    shows a schematic view of another preferred embodiment of the invention.

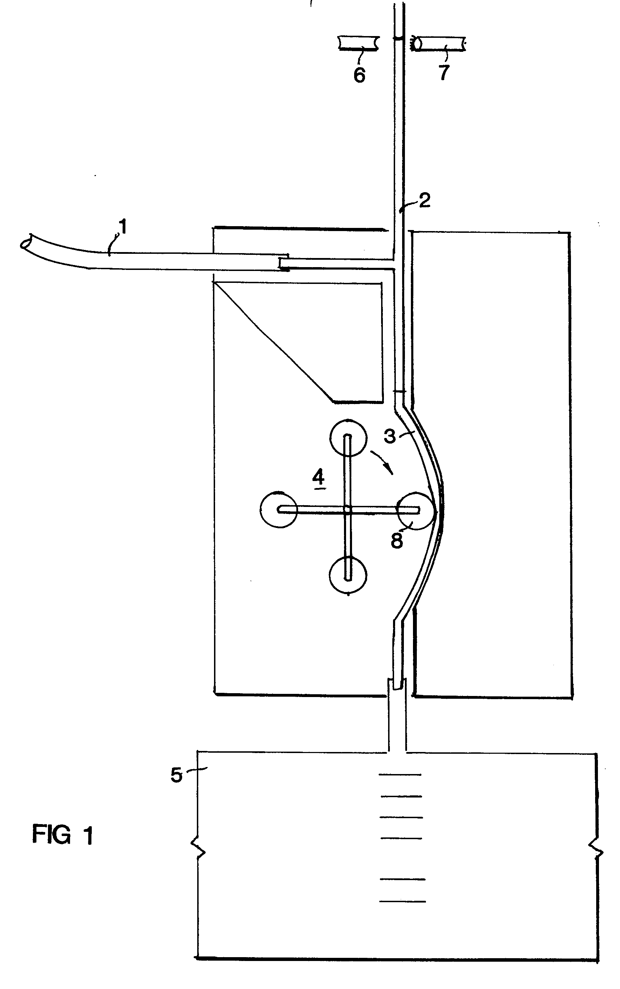

With reference to Fig 1, 1 denotes a tube connected to a catheter (not shown), which tube, in turn, is connected in its other end with a tube 2, suitably what is known as a T-tube, having a long superposed part, and whereby the T-tube is rotated 90°. The tube 2 can also be described as a vertically arranged tube having a connector in its side. The tube 2 is made of a translucent material, such as glass or glass clear polymer. The tube 2 is connected to a flexible tube 3 with its downwardly turned part, which flexible tube 3 is mounted in a peristaltic pump 4 and further connected to a urine collecting bag 5 of a type known per se. On each side of the tube 2, on the upper part thereof, a photocell 6, and a light source 7, respectively, are arranged.

The peristaltic pump 4 is of such a construction that it rotates a certain number of revolutions or part of a revolution, all in accordance with the basic adjustment thereof, when it receives a driving signal.

The device functions so that an amount of urine passes out through the urine catheter and into the tube 1, whereafter it passes into the tube 2. When this has been filled so high that the liquid column passes the light beam created by the light source 7 and received by the photocell 6 the light is broken and the photocell 6 gives an electrical signal which starts the driving motor of the peristaltic pump 4, whereby this goes for example 1/4 of a revolution, or ½ of a revolution, if so adjusted, whereby a certain, exact amount of urine is pumped out and through the flexible tube 3 to the urine bag 5. The photocell 6 will now receive light and the driving of the peristaltic pump is stopped; to be started again as soon as the light beam to teh photocell 6 is broken. The number of 1/4-revolutions or ½-revolutions which the pump makes can thereby automatically be registered from a set point of time, a zero-point, and thereby the volume of urine excreted can be carefully determined. The peristaltic pump 4 also presses so hard on the tube 3 that no leakage occurs downwardly behind the pressing roll 8 of the peristaltic pump.

It is evident to the one skilled in the art that the revolutions of the peristaltic pump 4 can be registered by a

4    0109373

computer unit (not shown), which can show the number of revolutions on a printer or a display  after the zero-point,
the number of revolutions during a first 10-minutes period,
during a later 10-minutes period, show volumes during certain time periods, accumulated volume and such other suitable and wanted information providing values. The time point
for each revolution of the peristaltic pump can thus be
registered and values obtained can be integrated and collected in any suitable way.

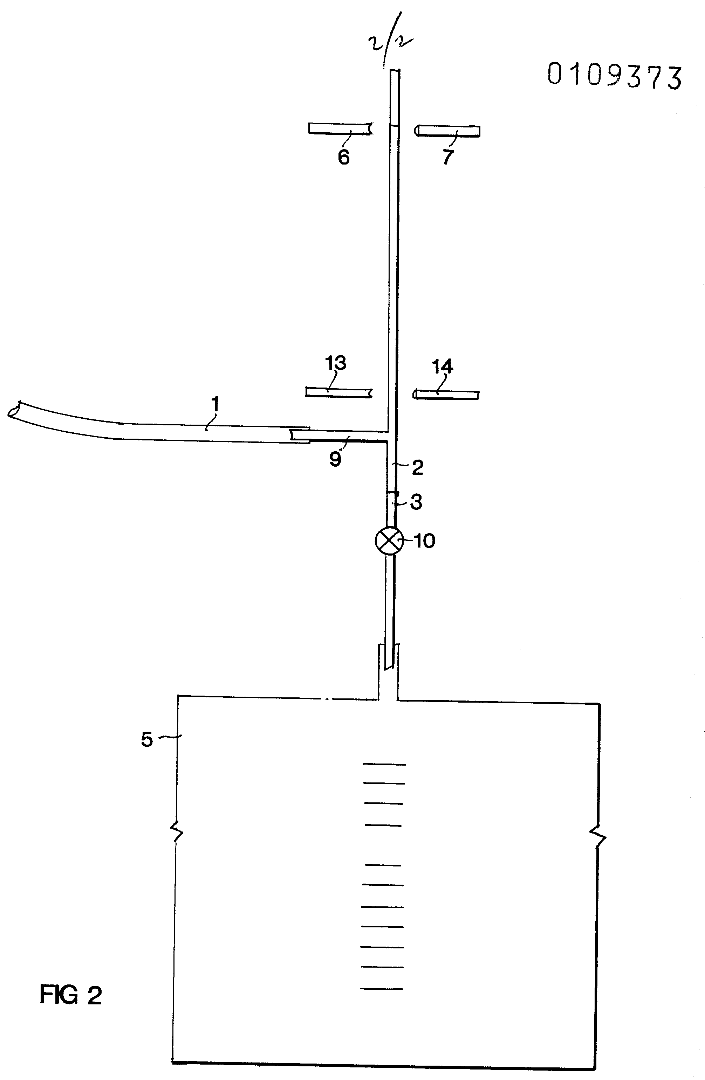

With reference to Fig 2 another preferred embodiment of the
invention, a tube 1 is shown, which in its one end is connected to a urine catheter and in its other end is connected to a vertically arranged tube 2 having a side connector
9, whereby connection has been made to said connector 9.
The tube 2 is downwardly connected by means of a tube 3 to
a urine collecting bag 5. The tube 3 is provided with a magnetic valve 10, which works by clamping the tube 3 against
a counter part 11 by means of a jaw 12 actuated by a magnet.
On each side of the upper part of the tube 2 are an upper
photocell 6, and an upper light source 7, respectively, and
and a lower photocell 13 and a lower light source 14, respectively, arranged.

This embodiment functions so that the magnetic valve 10
normally closes the tube 3, urine flows through the tube 1
and fills the tube 2. When the urine level reaches the upper photocell 6 the light beam directed thereto is broken,
a signal is transmitted to the magnetic valve 10, which
opens and urine flows downwardly to the urine bag 5. When
the urine level reaches the level for the lower photocell
13 this obtains a light beam from its light source 14,
whereby a signal is transmitted to the magnetic valve 10,
which closes the tube 3. Therby a volume of urine corresponding to the volume of the tube 2 between the photocells
6 and 13 has passed down to the bag 5, as well as a minor
amount directly from the tube 1 via the connector 9 and
downwardly. These values are, however, constant, and can
be easily determined, whereby the number of opening events of the magnetic valve directly corresponds to the volume of urine passed. In the same way, as mentioned above, the number of opening events of the magnetic valve can be determined and registered, i.e. the number of millilitres of urine excreted. The amount per given time unit as well as accumulated volume of urine for the total measuring period can be determined and calculated.

By connecting it to computer a guarding alarm can also be introduced quite simple. so that at a non-reaching of a given minimum volume an alarm is given to a centre being continously attended to as is the case on an intense care unit.

The computer use also means that values can be easily stored and be brought forward if so requested, even a long time after the patient has left the hospital.

The light used in the photocell/light source can be of a visible or non-visible wave-length. It can for some reason be necessary to utilize non-visible light, if one fears that strong light may affect the photocell. The photocell, can however, be considerably protected against infalling light by being placed in a cylinder, whereby only the light being directed from the light source affects it.

In one embodiment above the use of a magnetic valve has been shown. It is evident that any other type of valve can be used, which valve fulfills the requirements.

The pump 4 above shall preferably be provided with a stepmotor which drives a given number of steps when it obtains a signal. These steps can then easily be registered. This type of motor will also provide for a very accurate measurment.

The present device has shown to provide very accurate measurements in the range from 2- 18000 ml per 24 hours, and can thus be used in all types of situations.

**0109373**

CLAIMS.

1. Device for measuring urine excreted by a catheterized patient, which device comprises at least one photocell/ light source and an opening/closing means, characterized by a vertical tube (2) arranged to be connected to said catheter by means of a connector (9) arranged on its side; at least one photocell(6) and a light source (7) related to said photocell (6), respectively, which are arranged around the upper part of said tube (2) in order to register a reached level of collected urine in said tube (2); and an opening/closing means (4,10) for opening/closing of a lower outlet of said tube (2) or part (3) connected thereto, whereby said photocell/light source (6,7) is arranged to control the opening/closing means (4,10) for the elimination of the urine collected in said tube (2).

2. Device according to claim 1, characterized in that it comprises an opening/closing means in the form of a pump (4) controlled by said photocell/light source (6,7), which pump (4) is arranged to pump a given, predetermined amount of urine as an answer on a contolling signal from said photocell/light source (6,7).

3. Device according to claim 1, characterized in that the device comprises two photocells/light souces (6,7;13,14) placed on a distance from each other around the tube (2) on the part thereof situated above said connector (9), which photocells/light sources (6,7;13,14) control opening and closing, respectively, of the opening/closing means (10).

4. Device according to claims 1-4, characterized in that the number of controlling events of the opening/closing means (4,10) is arranged to be stored and calculated in a calculation unit provided with a memory, from which any calculations can be taken out for evaluation.

0109373

FIG 1

2/2

0109373

FIG 2